# EUROPEAN PATENT APPLICATION

(11) **EP 4 678 187 A1**
(43) Date of publication of application: **14.01.2026**
(21) Application number: 24188151.5
(22) Date of filing: 11.07.2024
(51) Int. Cl.: A61K 40/00, A61K 39/00, A61P 1/00, A61P 37/06, C07K 14/725, C07K 16/28, C07K 16/40

(54) **COMPOUND FOR USE IN THE TREATMENT OF INFLAMMATORY BOWEL DISEASE**

(71) Applicant: Medizinische Hochschule Hannover, 30625 Hannover (DE); Technische Universität Braunschweig, 38106 Braunschweig (DE)
(72) Inventor: JÄCKEL, Elmar, 30625 Hannover (DE); HARDTKE-WOLENSKI, Matthias, 30625 Hannover (DE); RIET, Tobias, 30625 Hannover (DE); KARSLI-ÜNAL, Ümran, 30625 Hannover (DE); HUST, Michael, 38106 Braunschweig (DE)
(74) Representative: Taruttis, Stefan Georg

(57) **Abstract**

The invention provides a fusion protein, a nucleic acid construct encoding the fusion protein and regulatory T-cells (Treg) expressing the fusion protein for use in the treatment of inflammatory bowel disease, wherein the fusion protein is a chimeric antigen receptor (CAR) having a single chain variable fragment (scFv) domain specific for being activated by the location of inflammatory bowel disease, especially binding to one of meprin 1α (MEP1A) and cadherin 17 (CDH17), a transmembrane domain and at least one intracellular signalling domain. The scFv domains specific for MEP1A or CDH17 have been found to selectively bind to intestine afflicted by inflammatory bowel disease and to activate suppressive activity in Treg expressing the CAR at the location of inflammatory bowel disease. Specifically, the invention provides a CAR for use in the treatment of an immune response, e.g. in the treatment of an autoimmune response, which is directed against MEP1A or directed against CDH17.

## Description

The present invention relates to a fusion protein suitable for use in the treatment of inflammatory bowel disease, which is a recurrent inflammation of the bowel, especially Morbus Crohn and Colitis ulcerosa. The invention has the advantage of generating an immune suppression which is essentially restricted to the location of the bowel inflammation, avoiding a generalized systemic suppression of the immune system.

Currently, the etiopathology of inflammatory bowel disease is not completely known, but genetic predisposition, environmental influences like food or stress, composition of the intestinal microflora and a systemic dysregulation of the immune system are assumed contributing factors.

### State of art

WO2013/153391 A1 and WO2021/239812 A1 describe suicide genes for use in genetically manipulated cells.

EP 3478710 B1describes the expression of a CAR which is specific for HLA*02 in regulatory T-cells for suppressing adverse immune reactions against cells bearing this cellular antigen.

### Object of the invention

It is an object of the invention to provide a compound for use in the treatment of inflammatory bowel disease, which compound preferably acts locally only without causing a systemic immune suppression. A further object is to provide a method for producing the compound for treatment of inflammatory bowel disease.

### Description of the invention

The invention achieves the object by the features of the claims, and especially by providing a fusion protein, a nucleic acid construct encoding the fusion protein and regulatory T-cells (Treg) expressing the fusion protein for use in the treatment of inflammatory bowel disease, wherein the fusion protein is a chimeric antigen receptor (CAR) having a single chain variable fragment (scFv) domain specific for being activated by the location of inflammatory bowel disease, especially binding to one of meprin 1α (MEP1A) and cadherin 17 (CDH17), a transmembrane domain and at least one intracellular signalling domain. The scFv domains specific for MEP1A or CDH17 have been found to selectively bind to intestine afflicted by inflammatory bowel disease and to activate suppressive activity in Treg expressing the CAR at the location of inflammatory bowel disease. Specifically, the invention provides a CAR for use in the treatment of an immune response, e.g. in the treatment of an autoimmune response, which is directed against MEP1A or directed against CDH17.

The CAR of the invention has an scFv which is specific for intestine afflicted by inflammatory bowel disease, the CAR when expressed in Treg resulting in generation of suppressive activity that is limited to the location of the inflammatory bowel disease, especially limited to the location of an acute phase of inflammatory bowel disease.

The CAR of the invention comprises or consists of a secretory signal domain, also termed leader, an scFv, an optional linker, a hinge domain, a transmembrane domain (TM) and at least one, preferably two intracellular signaling domains. These domains preferably are arranged directly adjacent to one another. The optional linker is arranged C-terminally adjacent to the scFv and N-terminally to the hinge. Preferably, the linker is a section of an antibody constant light chain (CL), or a section of an antibody constant heavy chain (CH1), in each case comprising or consisting of e.g. 10 to 18 amino acids, in each case ± 2 amino acids. In an embodiment, the CAR comprises a secretory signal domain, an scFv, optionally a linker, a hinge comprising or consisting of a IgG1 CH1 - IgG1 CH2 - IgG1 CH3 hinge, a TM which is a CD4 transmembrane domain or a CD4a transmembrane domain, and a CD28 signaling domain (CD28 ICD) and a CD3zeta signaling domain (CD3zeta ICD), which embodiment herein is also referred to as IgG-CAR. In another embodiment, the CAR comprises a secretory signal domain, an scFv, optionally a linker, a hinge comprising or consisting of a CD8 hinge, a TM which is a CD4 transmembrane domain or a CD4a transmembrane domain, and a CD28 signaling domain (CD28 ICD) and a CD3zeta signaling domain (CD3zeta ICD), which embodiment herein is also referred to as CD8-CAR.

Generally, the scFv domain comprises a variable heavy chain and a variable light chain, preferably a variable light chain (VL) of VL FR1 - VL CDR1 - VL FR2 - VL CDR2 - VL FR3 - VL CDR3 - VL FR4, and a variable heavy chain (VH) of VH FR1 - VH CDR1 - VH FR2 - VH CDR2 - VH FR3 - VH CDR3 - VH FR4. The CAR in its scFv domain has the CDR3 domains of both the variable light chain (VL) and of the variable heavy chain (VH), preferably the CDR1 domain, the CDR2 domain and the CDR3 domains of both the VL and VH, of one of SEQ ID NO: 1, SEQ ID NO: 3, SEQ ID NO: 5, SEQ ID NO: 7, SEQ ID NO: 9, SEQ ID NO: 11, SEQ ID NO: 13, and SEQ ID NO: 15. More preferably, the CAR contains one these scFv domains completely.

In an embodiment, the scFv is specific for binding MEP1A, the scFv having a amino acid sequence of
SEQ ID NO: 1 (MRU53-F8) 
QVQLQESGGGLVQPGRSLRLSCTASGFTFGDYAMSWVRQAPGKGLEWVGFIRSKAY
GGTTEYAASVKGRFTISRDDSKSIAYLQMNSLKTEDTAVYYCTRVGGPDYGDPFDY
WGQGTLVTVSSGSASAPKLEEGEFSEARVDIVMTQSPSSLSASVGDRATITCRASQSIN
NYLNWYQQKPGKAPNLLIYGASNLQGGVPSRFSGSGSGTDFTLTINSLQPEDFASYYC
QQTYNTPITFGQGTRLEII,
wherein preferably in the CAR, the scFv is comprised in SEQ ID NO: 2 
MKYLLPTAAAGLLLLAAQPAMAQVQLQESGGGLVQPGRSLRLSCTASGFTFGDYAM
SWVRQAPGKGLEWVGFIRSKAYGGTTEYAASVKGRFTISRDDSKSIAYLQMNSLKTE
DTAVYYCTRVGGPDYGDPFDYWGQGTLVTVSSGSASAPKLEEGEFSEARVDIVMTQ
SPSSLSASVGDRATITCRASQSINNYLNWYQQKPGKAPNLLIYGASNLQGGVPSRFSG
SGSGTDFTLTINSLQPEDFASYYCQQTYNTPITFGQGTRLEIIRTVAAPSVAA, which at
its N-terminus has a secretory signal domain and at its C-terminus has a linker of 10 amino acids,
or an amino acid sequence of SEQ ID NO: 3 (MRU53-H1) 
QVQLQQSGGGLVQPGRSLGLSCTASGFTFGDYAMSWVRQAPGKGLEWVGFIRSKAY
GGTTEYAASVKGRFTISRDDSESIAYLQMNSLKTEDTAVYYCTRGSYYSDAFDIWGQ
GTMVTVSSGSASAPKLEEGEFSEARVDIVLTQSPPSLSASVGDRVTITCRTSQSISSYLN
WYQQKPGKAPKLLIYGASSLQSGVPSRFSGSGSGTDFTLTISSLQPEDFATFYCQQSYS
SPPTFGQGTKVDIK,
wherein preferably in the CAR, the scFv is comprised in SEQ ID NO: 4 
MKYLLPTAAAGLLLLAAQPAMAQVQLQQSGGGLVQPGRSLGLSCTASGFTFGDYA
MSWVRQAPGKGLEWVGFIRSKAYGGTTEYAASVKGRFTISRDDSESIAYLQMNSLKT
EDTAVYYCTRGSYYSDAFDIWGQGTMVTVSSGSASAPKLEEGEFSEARVDIVLTQSP
PSLSASVGDRVTITCRTSQSISSYLNWYQQKPGKAPKLLIYGASSLQSGVPSRFSGSGS
GTDFTLTISSLQPEDFATFYCQQSYSSPPTFGQGTKVDIKRTVAAPSVAA
which at its N-terminus has a secretory signal domain and at its C-terminus has a linker of 10 amino acids,
or an amino acid sequence of SEQ ID NO: 5 (MRU54-A1) 
QVQLVQSGGGLVQPGRSLRLSCTASGFTFGDYAMSWVRQAPGKGLEWVGFIRSKAY
GGTTEYAASVKGRFTISRDDSKSIAYLQMNSLKTEDTAVYYCTRAVEMADAFDIWG
QGTMVTVSSGSASAPKLEEGEFSEARVDVVMTQSPSSLSASVGDRVTITCRASQSISS
YLNWYQQKPGKAPKLLIYAASSLQSGVPSRFSGSGSGTDFTLTISSLQPEDFATYYCQ
QSYSTPFTFGPGTKVDIK,
wherein preferably in the CAR, the scFv is comprised in SEQ ID NO: 6 
MKYLLPTAAAGLLLLAAQPAMAQVQLVQSGGGLVQPGRSLRLSCTASGFTFGDYA
MSWVRQAPGKGLEWVGFIRSKAYGGTTEYAASVKGRFTISRDDSKSIAYLQMNSLK
TEDTAVYYCTRAVEMADAFDIWGQGTMVTVSSGSASAPKLEEGEFSEARVDVVMT
QSPSSLSASVGDRVTITCRASQSISSYLNWYQQKPGKAPKLLIYAASSLQSGVPSRFSG
SGSGTDFTLTISSLQPEDFATYYCQQSYSTPFTFGPGTKVDIKRTVAAPSVAA
which at its N-terminus has a secretory signal domain and at its C-terminus has a linker of 10 amino acids.

In an embodiment, the scFv is specific for binding CDH17, the scFv having a amino acid sequence of
SEQ ID NO: 7 (ÜK013-B5, in the sequence protocol named UK013-B5) 
QVQLVQSGAEVRKSGESLKISCEGSGYSFGNYWIAWVRQMPGKGLEWMGIIYPGDS
DTRYSPSFQGQVTISADKSISTAYLQWNTLKASDTAMYFCARHVDSTWYIDHWGQG
TLVTVSSGSASAPKLEEGEFSEARVQPVLTQPPSASGSPGQSVTISCTGTSSDVGGYNF
VSWYQHHPGKAPKLMIYEVSKRPSGVPDRFSASKSGNTASLTVSGLQADDEADYYC
SSYAGNDNLQFGGGTKLTVL,
wherein preferably in the CAR, the scFv is comprised in SEQ ID NO: 8 
MKYLLPTAAAGLLLLAAQPAMAQVQLVQSGAEVRKSGESLKISCEGSGYSFGNYWI
AWVRQMPGKGLEWMGIIYPGDSDTRYSPSFQGQVTISADKSISTAYLQWNTLKASDT
AMYFCARHVDSTWYIDHWGQGTLVTVSSGSASAPKLEEGEFSEARVQPVLTQPPSAS
GSPGQSVTISCTGTSSDVGGYNFVSWYQHHPGKAPKLMIYEVSKRPSGVPDRFSASKS
GNTASLTVSGLQADDEADYYCSSYAGNDNLQFGGGTKLTVLGRPKAAPSVTLFPPSS
AA, which at its N-terminus has a secretory signal domain and at its C-terminus has a linker of 18 amino acids,
or an amino acid sequence of SEQ ID NO: 9 (ÜK013-D5, in the sequence protocol named UK013-D5) 
QVQLQQSGAEVKKPGESLKISCEGSGYSFSNYWIAWVRQMPGKGLEWMGVIYPGDS
DTRYSPSFQGQVTISADKSISTAYLQWSSLKASDTAMYYCARPPVGATDAFDIWGQG
TMVTVSSGSASAPKLEEGEFSEARVQSALTQPPSASGSPGQSVTISCTGTSSDVGGYNF
VSWYQQHPGKAPKLMIYEVSKRPSGVPDRFSGSKSGNTASLTVSGLQAEDEADYYCS
SYAGSNNYVFGTGTKVTVL,
wherein preferably in the CAR, the scFv is comprised in SEQ ID NO: 10, MKYLLPTAAAGLLLLAAQPAMAQVQLQQSGAEVKKPGESLKISCEGSGYSFSNYWI AWVRQMPGKGLEWMGVIYPGDSDTRYSPSFQGQVTISADKSISTAYLQWSSLKASD TAMYYCARPPVGATDAFDIWGQGTMVTVSSGSASAPKLEEGEFSEARVQSALTQPPS ASGSPGQSVTISCTGTSSDVGGYNFVSWYQQHPGKAPKLMIYEVSKRPSGVPDRFSGS KSGNTASLTVSGLQAEDEADYYCSSYAGSNNYVFGTGTKVTVLGQPKANPTVTLFPP SSAA, which at its N-terminus has a secretory signal domain and at its C-terminus has a linker of 18 amino acids,
or an amino acid sequence of SEQ ID NO: 11 (ÜK013-F8, in the sequence protocol named UK013-F8) 
QVQLQESGGGLVKPGGSLRLSCAASGFTFSSYSMNWVRQAPGKGLEWVSSISSSSYI
YYADSVKGRFTISRDNAKNSLYLQMNSLRAEDTAVYYCARREGGKYYYGMDVWG
QGTTVTVSSGSASAPKLEEGEFSEARVQSALTQPPSASGSPGQSVTISCTGTSSDVGAY
 
NYVSWYQQHPGKAPKLMIYEVTERPSGVPDRFSGSKSGNTASLTISGLQAEDEADYY
CSSYTTSSTVLFGGGTKLTVL,
wherein preferably in the CAR, the scFv is comprised in SEQ ID NO: 12, 
MKYLLPTAAAGLLLLAAQPAMAQVQLQESGGGLVKPGGSLRLSCAASGFTFSSYSM
NWVRQAPGKGLEWVSSISSSSYIYYADSVKGRFTISRDNAKNSLYLQMNSLRAEDTA
VYYCARREGGKYYYGMDVWGQGTTVTVSSGSASAPKLEEGEFSEARVQSALTQPPS
ASGSPGQSVTISCTGTSSDVGAYNYVSWYQQHPGKAPKLMIYEVTERPSGVPDRFSG
SKSGNTASLTISGLQAEDEADYYCSSYTTSSTVLFGGGTKLTVLGQPKAAPSVTLFPPS
SAA, which at its N-terminus has a secretory signal domain and at its C-terminus has a linker of 18 amino acids,
or an amino acid sequence of SEQ ID NO: 13 (ÜK013-H4, in the sequence protocol named UK013-H4) 
QVQLQESGGGVVQPGRSLRLSCAASGFTFSSYAMHWVRQAPGKGLEWVAVISYDGS
NKYYADSVKGRFTISRDNSKNTLYLQMNSLRAEDTAVYYCARAPYYYDTPRTYYYG
MDVWGQGTTVTVSSGSASAPKLEEGEFSEARVSYELTQPPSASGTPGQTFTISCSGSD
SNIERNFVYWYQQLPGTAPKLLIYRTDQRPSGVPDRFSGSKSGSSASLAISGLRSEDEA
HYYCSTWDDSLTGVVFGGGTKLTVL,
wherein preferably in the CAR, the scFv is comprised in SEQ ID NO: 14, MKYLLPTAAAGLLLLAAQPAMAQVQLQESGGGVVQPGRSLRLSCAASGFTFSSYAM HWVRQAPGKGLEWVAVISYDGSNKYYADSVKGRFTISRDNSKNTLYLQMNSLRAE DTAVYYCARAPYYYDTPRTYYYGMDVWGQGTTVTVSSGSASAPKLEEGEFSEARVS YEL TQPPSASGTPGQTFTISCSGSDSNIERNFVYWYQQLPGT APKLLIYR TDQRPSGVP DRFSGSKSGSSASLAISGLRSEDEAHYYCSTWDDSLTGVVFGGGTKLTVLGQPKAAP
SVTLFPPSSAA, which at its N-terminus has a secretory signal domain and at its C-terminus has a linker of 18 amino acids,
or an amino acid sequence of SEQ ID NO: 15 (ÜK027-F5, in the sequence protocol named UK027-F5) 
QVQLVQSGAEVKKPGASVKVSCKASGYTFTGYYMHWVRQAPGQGLEWMGWINPN
SGGTNYAQKFQGRVTMTRDTSISTAYMELSRLRSDDTAVYYCARAPFGSADYWGQG
TLVTVSSGSASAPKLEEGEFSEARVQPVLTQPPSVSVSPXQTASISCSGDRLGDKYVA
WYQQKAGQSPVLVIYADAKRPSGIPERFSGSNSGNTATLTISGTQALDEGDYYCQAW
DTFTVIFGGGTKLTVL,
wherein preferably in the CAR, the scFv is comprised in SEQ ID NO: 16 
MKYLLPTAAAGLLLLAAQPAMAQVQLVQSGAEVKKPGASVKVSCKASGYTFTGYY
MHWVRQAPGQGLEWMGWINPNSGGTNYAQKFQGRVTMTRDTSISTAYMELSRLRS
DDTAVYYCARAPFGSADYWGQGTLVTVSSGSASAPKLEEGEFSEARVQPVLTQPPSV
SVSPXQTASISCSGDRLGDKYVAWYQQKAGQSPVLVIYADAKRPSGIPERFSGSNSGN
TATLTISGTQALDEGDYYCQAWDTFTVIFGGGTKLTVLGQPKAAPSVTLFPPSSAA,
which at its N-terminus has a secretory signal domain and at its C-terminus has a linker of 18 amino acids.

Preferably the scFv comprises or consists of one of SEQ ID NO: 1, SEQ ID NO: 3 and SEQ ID NO: 5, SEQ ID NO: 7, SEQ ID NO: 9, SEQ ID NO: 11, SEQ ID NO: 13, and SEQ ID NO: 15, more preferably, the scFv domain with an N-terminal secretory signal and a C-terminal linker comprises or consists of one of SEQ ID NO: 2, SEQ ID NO: 4 and SEQ ID NO: 6, SEQ ID NO: 8, SEQ ID NO: 10, SEQ ID NO: 12, SEQ ID NO: 14, and SEQ ID NO: 16.

Generally preferred, the Treg expressing a CAR of the invention are immunologically compatible to the recipient, and preferably the Treg originate from T cells originating from the recipient, i.e. the Treg are derived from autologous T cells, wherein they are derived by genetic manipulation to express the CAR, preferably in combination with FOXP3, and optionally express the peptide label, which preferably is CD20 or deltaLNGFR for use in humans, or Thy 1.1 for use in mice.

The secretory signal peptide can e.g. have an amino acid sequence as contained in one of SEQ ID NO: 2, NO: 4, NO: 6, NO: 8, NO: 10, NO: 12, NO: 14, and NO: 16, or
MDFQVQIFSFLLISASVIMSRT (SEQ ID NO: 17, murine) or
MWWRLWWLLLLLLLLWPMVWA (SEQ ID NO: 18, murine), preferably
MWWRLWWLLLLLLLLWPMVW (SEQ ID NO: 19, human), or
MDFQVQIFSFLLISASVIMSR (SEQ ID NO: 20, human).

The hinge domain, which connects the scFv portion with the transmembrane domain, can e.g. have an amino acid sequence comprising or consisting of the IgG1 hinge, the IgG1 CH2 hinge and the IgG1 CH3 hinge, the hinge region of CD28, of Cd8α, of CD4, of CD7, of CH2CH3, of an immunoglobulin, or a part or variant thereof, preferably the CD8α hinge domain or CH2CH3 hinge domain, all of human origin or all of murine origin, e.g. 
VPRDGGCKPCICTVPEVSSVFIFPPKPKDVLTITLTPKVTCVVVDISKDDPEVQFSWFV
DDVEVHTAQTQPREEQFNSTFRSVSELPIMHQDWLNGKEFKCRVNSAAFPAPIEKTIS
KTKGRPKAPQVCTIPPPKEQMAKDKVSLTCMITDFFPEDITVEWQWNGQPAENYKNT
QPIMDTDGSYFVYSKLNVQKSNWEAGNTFTCSVLHEGLHNHHTEKSLSHS
 PG (SEQ ID NO: 21, murine) which consists of the mIgG1 hinge, the mIgG1 CH2 hinge and the mIgG1 CH3 hinge, or the CD8 hinge of human origin or of murine origin, e.g. FSSVVPVLQKVNSTTTKPVLRTPSPVHPTGTSQPQRPEDCRPRGSVKGTGLDFACDIY (SEQ ID NO: 22, murine), or e.g. FVPVFLPAKPTTTPAPRPPTPAPTIASQPLSLRPEASRPAAGGAVHTRGLDFADIYIWAP LAGTCGVLLLSLVITLYCNHRQL (SEQ ID NO: 23, human) consisting of the hCD8 hinge and the hCD28 transmembrane domain (hCD8 TM), or the deltaFc Ig, which in the CAR has the functions of a hinge domain and a transmembrane domain, e.g. having the amino acid sequence 
PAEPKSPDKTHTCPPCPAPPVAGPSVFLFPPKPKDTLMIARTPEVTCVVVDVSHEDPEV
KFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCSV
MHEALHNHYTQKSLSLSPGKK (SEQ ID NO: 24, human).
 A transmembrane domain may be selected from the transmembrane domains of CD28, of ICOS, of CD8α, of CD4, of CD134 (OX40), of CD137 (4-1BB), of CD3 zeta, of CD45, of CD9, of CD16, of CD22, of CD33, of CD64, of CD80, of CD86, of CD154, of CH2CH3, or a part or variant thereof. Preferably, the CAR comprises a transmembrane domain of CD8α or of CH2CH3. As examples, the transmembrane domain (TM) may be the CD4 TM, e.g. VFLACVLGGSFGFLGFLGLCILCCV (SEQ ID NO: 25, murine) or its human equivalent, or the CD8a transmembrane domain CD8a TM, e.g. IWAPLAGICVALLLSLIITLICYHR (SEQ ID NO: 26, murine) or its human equivalent.

Preferably, the intracellular signaling domains comprise or consist of, from N-terminus to C-terminus, the CD28 signaling domain (CD28 ICD) and the CD3zeta signaling domain (CD3z ICD). The CD28 ICD can e.g. have the amino acid sequence QLWTNSRRNRLLQSDYMNMTPRRPGLTRKPYQPYAPARDFAAYRP (SEQ ID NO: 27, murine) and the CD3z ICD can e.g. have the amino acid sequence 
RAKFSRSAETAANLQDPNQLYNELNLGRREEYDVLEKKRARDPEMGGKQQRRRNPQ
EGVYNALQKDKMAEAYSEIGTKGERRRGKGHDGLYQGLSTATKDTYDA
 LHMQTLAPR (SEQ ID NO: 28, murine), or the CD28 ICD can e.g. have the amino acid sequence WTNSRRNRLLQSDYMNMTPRRPGLTRKPYQPYAPARDFAAYRP (SEQ ID NO: 29, murine), the CD3z ICD can e.g. have the amino acid sequence

RAKFSRSAETAANLQDPNQLYNELNLGRREEYDVLEKKRARDPEMGGKQQRRRNPQ
EGVYNALQKDKMAEAYSEIGTKGERRRGKGHDGLYQGLSTATKDTYDALHMQTLA
PR (SEQ ID NO: 30, murine).

The human CD28 ICD can e.g. have the amino acid sequence WVRSKRSRLLHSDYMNMTPRRPGPTRKHYQPYAPPRDFAAYRS (SEQ ID NO: 31, human), the human CD3z can have the amino acid sequence RVKFSRSADAPAYQQGQNQLYNELNLGRREEYDVLDKRRGRDPEMGGKPRRKNPQ EGLYNELQKDKMAEAYSEIGMKGERRRGKGHDGLYQGLSTATKDTYDA LHMQALPPRSSR (SEQ ID NO: 32, human), or the human CD28 ICD can have the amino acid sequence DPKFWVLVVVGGVLACYSLLVTVAFIIFWVRSKRSRLLHSDYMNMTPRRPGPTRKH YQAYAAARDFAAYRSL (SEQ ID NO: 33, human), and the human CD28 ICD can have the amino acid sequence

RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDA
LHMQALPPR (SEQ ID NO: 34, human).

The CAR preferably has an intracellular domain (ICD) comprising or consisting of one or more intracellular signaling domains selected from the group consisting of the CD3zeta signaling domain or any of its homologs, the signaling domain of a CD3 polypeptide, of a syk family tyrosine kinase, of a src family tyrosine kinase, of CD2, of CD5, and of CD8, or a signaling domain of a part or variant thereof. Preferably, the CAR comprises the CD3 zeta signaling domain. Generally preferred, the CAR comprises or consists of a human signal peptide, an scFv, human a CD28 hinge and a human CD28 transmembrane domain, a human CD28 ICD and a human CD3z ICD, or the CAR comprises or consists of a human signal peptide, an scFv, a human delta Fc Ig as a hinge and transmembrane domain, a human CD28 ICD and a human CD3z ICD, especially for use in the treatment of adverse immune reactions directed against the intestine in a human, especially for use in the treatment of inflammatory bowel disease.

The CAR may comprise one or more co-stimulatory domains. The one or more co-stimulatory domains may be selected from the intracellular domains of CD28, of ICOS, of CD134 (OX40), of CD137 (4-1BB), of CD27, or of TNFRSF25, or of a part or variant thereof. Preferably, the CAR may comprise a CD28 co-stimulatory domain. The one or more co-stimulatory domain can be arranged C-terminally to the transmembrane domain.

The CAR, especially the intracellular domains, also referred to as endodomains, of the CAR which are arranged C-terminally to the transmembrane domain, may comprise one or more intracellular signaling domains selected from the group consisting of the CD3 zeta signaling domain or any of its homologs, a CD3 polypeptide signaling domain, a syk family tyrosine kinase signaling domain, a src family tyrosine kinase signaling domain, CD2 signaling domain, CD5 signaling domain, and CD8 signaling domain, or a part or variant thereof. Preferably, the CAR may comprise the CD3zeta signaling domain.

The CAR may comprise one or more co-stimulatory domains, e.g. arranged C-terminally to the transmembrane domain of the CAR. The one or more co-stimulatory domains may be selected from the intracellular domains of CD28, of ICOS, of CD134 (OX40), of CD137 (4-1BB), of CD27, or of TNFRSF25, or a part or variant thereof. Preferably, the CAR may comprise a CD28 co-stimulatory domain.

The CAR preferably comprises or consists of a signal peptide, an scFv, a hinge comprising Ig G1 hinge, Ig G1 CH2 hinge and Ig G1 CH3 hinge, a CD4 transmembrane domain, a CD28 ICD and a CD3zeta ICD, or the CAR comprises or consists of a signal peptide, an scFv, a CD8 hinge, a CD8a transmembrane domain, a CD28 ICD and a CD3z ICD, for use in humans in each case preferably all of human origin, for use in mice preferably all of murine origin. The CAR is preferably encoded as a fusion protein, in which C-terminally to the CAR, a protease site, e.g. P2A, and FOXP3 are linked. The P2A protease site can have the amino acid sequence ATNFSLLKQAGDVEENPGP (SEQ ID NO: 35), FOXP3 can have the amino acid sequence

MPNPRPAKPMAPSLALGPSPGVLPSWKTAPKGSELLGTRGSGGPFQGRDLRSGAHTS
SSLNPLPPSQLQLPTVPLVMVAPSGARLGPSPHLQALLQDRPHFMHQL
STVDAHAQTPVLQVRPLDNPAMISLPPPSAATGVFSLKARPGLPPGINVASLEWVSRE
PALLCTFPRSGTPRKDSNLLAAPQGSYPLLANGVCKWPGCEKVFEEPEE

FLKHCQADHLLDEKGKAQCLLQREVVQSLEQQLELEKEKLGAMQAHLAGKMALAK
APSVASMDKSSCCIVATSTQGSVLPAWSAPREAPDGGLFAVRRHLWGSH
GNSSFPEFFHNMDYFKYHNMRPPFTYATLIRWAILEAPERQRTLNEIYHWFTRMFAY
FRNHPATWKNAIRHNLSLHKCFVRVESEKGAVWTVDEFEFRKKRSQRP
NKCSNPCP (SEQ ID NO: 36, murine) or

ATNFSLLKQAGDVEENPGPSMPNPRPAKPMAPSLALGPSPGVLPSWKTAPKGSELLG
TRGSGGPFQGRDLRSGAHTSSSLNPLPPSQLQLPTVPLVMVAPSGARLGPSPHLQALL
QDRPHFMHQLSTVDAHAQTPVLQVRPLDNPAMISLPPPSAATGVFSLKARPGLPPGI
NVASLEWVSREPALLCTFPRSGTPRKDSNLLAAPQGSYPLLANGVCKWPGCEKVFEE
PEEFLKHCQADHLLDEKGKAQCLLQREVVQSLEQQLELEKEKLGAMQAHLAGKMA
LAKAPSVASMDKSSCCIVATSTQGSVLPAWSAPREAPDGGLFAVRRHLWGSHGNSSF
PEFFHNMDYFKYHNMRPPFTYATLIRWAILEAPERQRTLNEIYHWFTRMFAYFRNHP
ATWKNAIRHNLSLHKCFVRVESEKGAVWTVDEFEFRKKRSQRPNKCSNPCP (SEQ ID
NO: 37, murine).

Preferably FOXP3 (human) has an amino acid sequence comprising or consisting of








 (SEQ ID NO: 38).

Generally, the CAR is expressed in Treg which are genetically manipulated to contain a nucleic acid construct containing an expression cassette encoding the CAR.

Accordingly, the invention provides Treg which are genetically manipulated to express at least one CAR of the invention, wherein the Treg are e.g. genetically manipulated to contain a nucleic acid construct containing an expression cassette encoding the CAR. In an embodiment, the Treg are genetically manipulated to express at least one CAR having an scFv specific for MEP1A and at least one CAR specific for CDH17.

Generally herein, amino acid sequences and the arrangement of elements of proteins are described from N-terminus to C-terminus, nucleic acid sequences are described from 5' to 3'. The elements, e.g. domains, of the CAR preferably are all of murine origin for use in the treatment of in mice, or all of human origin, especially for use in the treatment of humans.

Preferably, the CAR of the invention is encoded by a nucleic acid construct and in 3' to the coding sequence there is arranged a coding sequence for a protease site, e.g. a P2A site, and a coding sequence for FOXP3, such that the CAR is translated from one common transcript which encodes the protease site and FoxP3. Further preferred, the nucleic acid construct includes, e.g. in 3' to the coding sequence for FOXP3, an IRES (internal ribosome entry site) followed by a coding sequence for a reporter or suicide peptide, e.g. Thy1.1 or deltaLNGFR.

In an embodiment in which Treg express the CAR in combination with a suicide gene, the Treg are suitable for use in the treatment of adverse immune reactions directed against MEP1A and/or CDH17, with the additional feature of terminating the activity of the Treg, e.g. deleting the Treg, by using an antibody specific for the marker peptide, herein represented by deltaLNGFR for use in humans, or Thy1.1 for use in mice, which represents a suicide moiety for binding of an antibody.

The safety switch polypeptide provides a cell in or on which it is expressed with a suicide moiety. This is useful as a safety mechanism which allows a cell which has been administered to a subject to be deleted should the need arise, or indeed more generally, according to desire or need, for example once a cell has performed or completed its therapeutic effect.

A suicide moiety possesses an inducible capacity to lead to cellular death, or more generally to elimination or deletion of a cell. An example of a suicide moiety is a suicide protein, encoded by a suicide gene, which may be expressed in or on a cell alongside a desired transgene, in this case the CAR, which when expressed allows the cell to be deleted to turn off expression of the transgene (CAR). A suicide moiety herein is a suicide polypeptide that is a polypeptide that under permissive conditions, namely conditions that are induced or turned on, is able to cause the cell to be deleted.

The suicide moiety may be a polypeptide, or amino acid sequence, which may be activated to perform a cell-deleting activity by an activating agent which is administered to the subject, or which is active to perform a cell-deleting activity in the presence of a substrate which may be administered to a subject. In a particular embodiment, the suicide moiety may represent a target for a separate cell-deleting agent which is administered to the subject. By binding to the suicide moiety, the cell-deleting agent may be targeted to the cell to be deleted. In particular, the suicide moiety may be recognised by an antibody, and binding of the antibody to the safety switch polypeptide, when expressed on the surface of a cell, causes the cell to be eliminated, or deleted.

The suicide moiety may be HSV-TK or iCasp9. However, it is preferred for the suicide moiety to be, or to comprise, an epitope which is recognised by a cell-deleting antibody or other binding molecule capable of eliciting deletion of the cell. In such an embodiment, the safety switch polypeptide is expressed on the surface of a cell. The term "delete" as used herein in the context of cell deletion is synonymous with "remove" or "ablate" or "eliminate". The term is used to encompass cell killing, or inhibition of cell proliferation, such that the number of cells in the subject may be reduced. 100% complete removal may be desirable but may not necessarily be achieved. Reducing the number of cells, or inhibiting their proliferation, in the subject may be sufficient to have a beneficial effect.

In particular, the suicide moiety may be a CD20 epitope which is recognised by the antibody Rituximab. Thus, in the safety switch polypeptide the suicide moiety may comprise a minimal epitope based on the epitope from CD20 that is recognised by the antibody Rituximab. Biosimilars for Rituximab are available and may be used. A person of skill in the art is readily able to use routine methods to prepare an antibody having the binding specificity of Rituximab using the available amino acid sequences therefor.

CAR-cells specific for ASGPR, which also express a safety switch polypeptide comprising this sequence can be selectively killed using the antibody Rituximab, or an antibody having the binding specificity of Rituximab. The safety switch polypeptide is expressed on the cell surface and when the expressed polypeptide is exposed to or contacted with Rituximab, or an antibody with the same binding specificity, death of the cell ensues.

Thus, Rituximab, or an antibody having the binding specificity thereof, may be provided for use in adoptive cell transfer (ACT) in combination with a cell of the invention. The cell or nucleic acid or vector or construct for production of the cell and the Rituximab or equivalent antibody may be provided in a kit, or as a combination product.

For example, the suicide constructs of WO2013/153391 or WO2021/239812 may be used in a cell or cell population (e.g., Treg or Treg population) as described herein.

For the CAR, a preferred signal domain is a secretory signal domain, also designated secretory signal.

A preferred hinge domain is one of a CD8 hinge and a deltaFc IgG hinge, a preferred transmembrane domain (TM) is one of a CD8 TM or a CD4 TM, preferably in an arrangement of the CD8 hinge with the CD8 TM, or the deltaFc IgG hinge with the CD4 TM.

The intracellular signalling domains (ICD) are one or both of a CD28 ICD and CD3zeta ICD, preferably the CD28 ICD and the CD3zeta ICD.

Generally, for use in medical treatment, the CAR is expressed preferably in regulatory T-cells (Treg), characterized by the markers CD4⁺ CD25⁺CD127^{low}. Preferably, the regulatory T-cells originate from the human to be treated who, after genetic manipulation of the T-cells such that they express the CAR, can receive the regulatory T-cells expressing the CAR. Especially for use in medical treatment, the CAR in addition to the scFv contains human domains, e.g. as encoded by SEQ ID NO: 39 or SEQ ID NO: 40. For use in mice, e.g. for use in research, the CAR in addition to the scFv preferably contains murine domains, e.g. as encoded by SEQ ID NO: 41 or SEQ ID NO: 42.

Cloning of murine and human scFvs into murine and human retroviral vectors, respectively, was performed to generate CARs with specificity for MEP1A or for CDH17. The CARs either had a murine CD8α hinge and transmembrane domain (short hinge) or a murine IgG hinge domain and CD4 transmembrane domain (long hinge). Retroviral vectors containing LTR flanked 2nd generation CAR scaffolds were used for cloning. Cloning was performed by digestion of vectors with Ncol/Notl restriction enzymes and subsequent ligation of the scFv encoding nucleic acid sequence into the CAR backbone. Murine CAR backbones contained an additional Foxp3 expression cassette allowing converted Tregs (cTreg) generation out of CD4+ T cells but lacked such a cassette for generation of CAR Teffs or natural Tregs (nTregs). Vectors also contained Thy1.1. (CD90.1) as a CAR expression marker.

Production of recombinant CAR expressing retroviral virus and transduction of T cells and cell lines:
Retroviral particles allowing for CAR transduction of T cells were produced in HEK293T cells. They were K73 ecotropic-pseudotyped for murine constructs and VSV-G pantropic-pseudotyped for human constructs, respectively. For CAR transduction, CD4+ cells were enriched from murine spleenocytes by magnetic bead separation and activated by addition of anti-CD3/anti-CD28 beads for 2 days, followed by spin-transduction with virus particles and protaminsulfate and then underwent continued cultivation for 1 - 4 days. For a CAR activation assay, NFAT-GFP reporter murine T cell hybridoma cells were transduced in an analogous manner.

Hybridoma NFAT activation assay:
Murine T cell hybridoma cell line reporting NFAT activation by GFP expression were transduced with CAR vector as described above. In parallel, hepatocytes were purified that all express the target antigen, MEP1A or CDH17. After 48 h both cells of the cell line and hepatocytes were cultivated for 24 h to allow for CAR activation. In a parallel approach, MEP1A or CDH17 peptide (human or murine) that was directly coated on wells of a microtiter plate was used to stimulate CAR transduced hybridoma cells. Wells that were directly coated with collagenase protein and untreated wells served as controls. Cells were stained with anti-Fab antibody binding to the CAR domains for detecting CAR expression. The level of CAR activation reported by GFP and CAR expression was measured by flow cytometry. Herein, this T cell hybridoma cell line is also referred to as the reporter cell line or as reporter cells, which is used in the examples, unless indicated otherwise.

The invention is now described with reference to the figures, which show in
- Figs. 1A and 1B flow cytometry results showing activation of Treg cells without CAR (not transduced),
- Figs. 1C and 1D flow cytometry results showing activation of Treg cells expressing a CAR having the scFv MRU54-F8 as an IgG-CAR in presence of Caco-2 cells not expressing MEP1A (Caco-2 not transfected) or in presence of Caco-2 cells expressing human MEP1A (Caco-2 transfected with hMEP1A),
- Figs. 1E and 1F flow cytometry results showing activation of Treg cells expressing a CAR having the scFv MRU54-F8 as a CD8-CAR in presence of Caco-2 cells not expressing MEP1A (Caco-2 not transfected) or in presence of Caco-2 cells expressing human MEP1A (Caco-2 transfected with hMEP1A),
- Figs. 2A and 2B flow cytometry results showing activation of Treg cells without CAR (not transduced),
- Figs. 2C and 2D flow cytometry results showing activation of Treg cells expressing a CAR having the scFv MRU54-F8 as an IgG-CAR in presence of immobilized human MEP1A (immobilized hMEP1A) or in presence of phosphate-buffered saline (PBS) as a negative control,
- Figs. 2E and 2F flow cytometry results showing activation of Treg cells expressing a CAR having the scFv MRU54-F8 as a CD8-CAR in presence of immobilized human MEP1A (immobilized hMEP1A) or in presence of phosphate-buffered saline (PBS) as a negative control,
- Fig. 3 summarizes the activation of Treg transduced for expression of each of the CAR molecules of the invention in presence of, from left to right, PBS as negative control (1), non-transduced Caco2 cells as second negative control (2), Caco2 cells transfected for expressing human MEP1A (3), and for immobilized recombinant human MEP1A (5). The fourth and fifth bar graph groups represent the data of Figures 1 and 2,
- Figs. 4A and 4B flow cytometry results showing activation of Treg cells without CAR (not transduced) in presence of HEK293T cells not expressing CDH17 (HEK293T not transfected) or in presence of HEK293T cells expressing human CDH17 (HEK293T transfected with hCDH17),
- Figs. 4C and 4D flow cytometry results showing activation of Treg cells expressing a CAR having the scFv ÜK013-H4 as an IgG-CAR (ÜK013-H4+IgG-CAR) in presence of HEK293T cells not expressing CDH17 (HEK293T not transfected) or in presence of HEK293T cells expressing human CDH17 (HEK293T transfected with hCDH17),
- Figs. 4E and 4F flow cytometry results showing activation of Treg cells expressing a CAR having the scFv ÜK013-H4 as a CD8-CAR (ÜK013-H4+CD8-CAR) in presence of in presence of HEK293T cells not expressing CDH17 (HEK293T not transfected) or in presence of HEK293T cells expressing human CDH17 (HEK293T transfected with hCDH17),
- Figs. 5A and 5B flow cytometry results showing activation of Treg cells without CAR (not transduced) in presence of HEK293T cells expressing human CDH17 (HEK293T transfected with hCDH17) or in presence of immobilized human CDH17 (hCDH17),
- Figs. 5C and 5D flow cytometry results showing activation of Treg cells expressing a CAR having the scFv ÜK013-H4 as an IgG-CAR in presence of HEK293T cells expressing human CDH17 (HEK293T transfected with hCDH17) or in presence of immobilized human CDH17 (hCDH17),
- Figs. 5E and 5F flow cytometry results showing activation of Treg cells expressing a CAR having the scFv ÜK013-H4 as a CD8-CAR in presence of HEK293T cells expressing human CDH17 (HEK293T transfected with hCDH17) or in presence of immobilized human CDH17 (hCDH17),
- Fig. 6 shows background expression and background activation in medium, in Fig. 6A for Treg not transfected for expression of a CAR (Not transduced), Fig. 6B for Treg transfected for expression of scFv ÜK013-H4 as an IgG-CAR (ÜK013-H4+IgG-CAR), Fig. 6C for expression of scFv ÜK013-H4 as a CD8-CAR (ÜK013-H4+CD8-CAR), and
- Fig. 7 summarizes the activation of Treg transduced for expression of each of the CAR molecules of the invention in presence of, from left to right, PBS as negative control (1), non-transduced HEK293T cells as second negative control (2), HEK293T cells transfected for expressing human CDH17 (3), HEK293T cells transfected for expressing murine CDH17 (4), and for immobilized recombinant human CDH17 (5). The eighth and ninth bar graph groups represent the data of Figures 4, 5, and 6.

As the expression of the CAR in Treg cells after transfection by a viral particle containing a nucleic acid construct encoding an expression cassette for one of the CAR molecules of the invention depends on the efficacy of the transfection, and also the expression of the target antigen MEP1A or CHD17 in transfected cells, herein HEK293T cells or Caco-2 cells, depends on the efficacy of the transfection, the examples have been conducted for each MEP1A or CHD17 using aliquots of the same batch of Treg cells and of HEK293T cells or Caco-2 cells. Accordingly, the data shown in the Figures result from parallel assays conducted under the same conditions by the same technicians and therefore allow for comparison of expression and activation, separately for each of MEP1A and CHD17.

In the examples, the following CARs are used:
Secretory signal - scFv of SEQ ID NO: 1 (MRU53-F8) as a human IgG-CAR (MRU53-F8+IgG-CAR) encoded by SEQ ID NO: 39,
secretory signal - scFv of SEQ ID NO: 1 (MRU53-F8) as a human CD8-CAR (MRU53-F8+CD8-CAR) encoded by SEQ ID NO: 40,
secretory signal - scFv of SEQ ID NO: 3 (MRU53-H1) as an IgG-CAR (MRU53-H1+IgG-CAR) and as an IgG-CAR (MRU53-H1+IgG-CAR),
secretory signal - scFv of SEQ ID NO: 5 (MRU53-A1) as an IgG-CAR (MRU53-A1+IgG-CAR) and as an IgG-CAR (MRU53-A1+IgG-CAR),
secretory signal - scFv of SEQ ID NO: 7 (ÜK013-B5) as an IgG-CAR (ÜK013-B5+IgG-CAR) and as an IgG-CAR (ÜK013-B5+IgG-CAR),
secretory signal - scFv of SEQ ID NO: 9 (ÜK013-D5) as an IgG-CAR (ÜK013-D5+IgG-CAR) and as an IgG-CAR (ÜK013-D5+IgG-CAR),
secretory signal - scFv of SEQ ID NO: 11 (ÜK013-F8) as an IgG-CAR (ÜK013-F8+IgG-CAR) encoded by SEQ ID NO: 42, and as an CD8-CAR (ÜK013-F8+CD8-CAR) encoded by SEQ ID NO: 41,
secretory signal - scFv of SEQ ID NO: 13 (ÜK013-H4) as an IgG-CAR (ÜK013-H4+IgG-CAR) and as an IgG-CAR (ÜK013-H4+IgG-CAR).

Generally, in the Figures showing FACS-plots, the X-axis, labelled GFP expression, indicates activation of Treg cells, and the Y-axis, labelled CAR expression, indicates extracellular presence of the CAR molecule indicated.

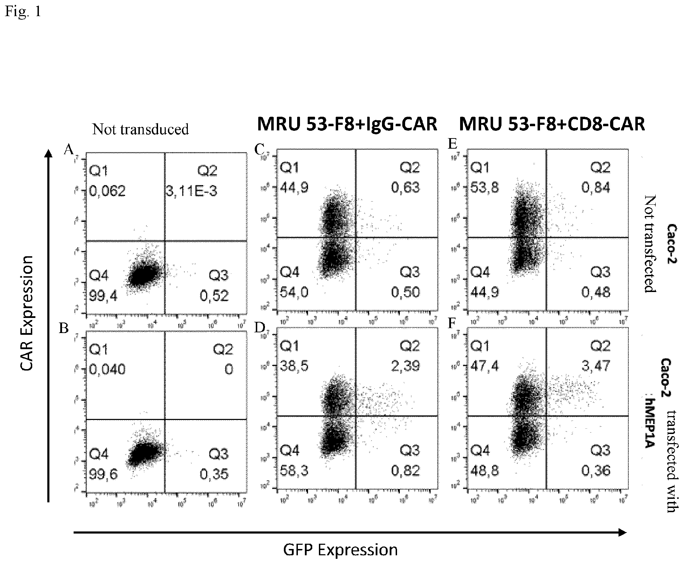

As a representative of a CAR having an scFv specific for MEP1A, a CAR having the scFv MRU53-F8 as an IgG-CAR and as a CD8-CAR, FACS-plots are shown in Fig. 1. Background detection in reporter cells which were not transduced with a nucleic acid construct encoding a CAR is shown by Figs. 1A and 1B. Fig. 1C and Fig. 1E show that both the IgG-CAR and the CD8-CAR embodiments of the CAR containing the scFv MRU53-F8 are expressed, and Figs. 1D and 1F show that in presence of Caco-2 cells expressing the target antigen hMEPIA the cells expressing the CAR are activated as detected by expression of GFP (GFP Expression) in the reporter cells. The antigen-specific activation is especially shown by Q2, when compared between Fig. 1C (Q2 = 0.63%) without target antigen expressed on cells and Fig. 1D (Q2 = 2.39%) in presence of target antigen expressed on cells, as well as between Fig. 1E (Q2 = 0.84%) without target antigen expressed on cells and Fig. 1F (Q2 = 3.47%) in presence of target antigen expressed on cells.

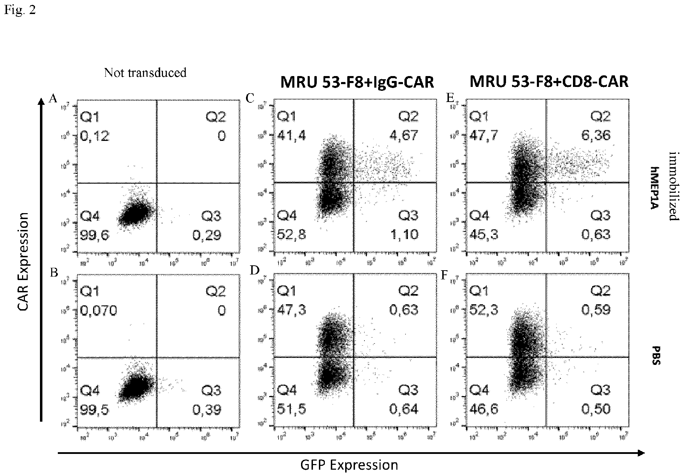

Fig. 2A and Fig. 2B show background of reporter cells which were not transduced with a nucleic acid construct encoding a CAR. Fig. 2D and Fig. 2F show that both the IgG-CAR and the CD8-CAR embodiments of the CAR containing the scFv MRU53-F8 are expressed without presence of target antigen but with phosphate buffered saline (PBS) as a negative control. Fig. 2C and Fig. 2E show that both embodiments of the CAR result in activation of the reporter cells by human MEP1A as the target antigen when immobilized on the culture plate (immobilized hMEP1A).

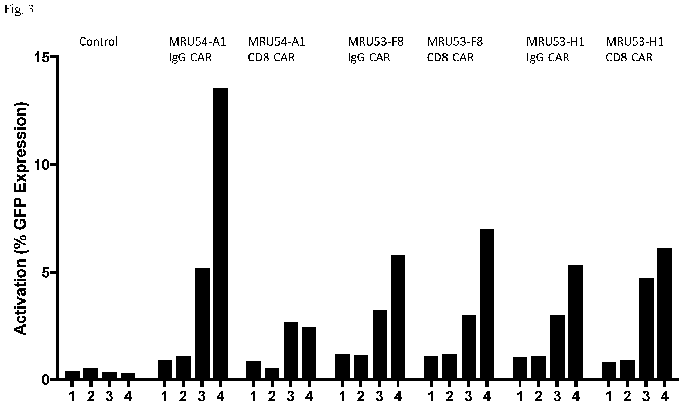

It is emphasized that the expression levels and activation levels for the MEP1 A-specific CARs or for the CDH17-specific CARs were measured in parallel experiments using aliquots of the same batch of cells in each case, allowing for a comparison of results as experimental variations between measurements, e.g. due to different cell cultures, different transfection conditions, different FACS calibrations or between different persons conducting the experiments were minimized. Fig. 3 summarizes background levels and activation levels of reporter cells expressing a CAR as indicated. All CARs of the invention show low background activation in the absence of the target antigen, and all CARs show high activation by presence of the target antigen when expressed on cells or when bound as protein to the culture plate.

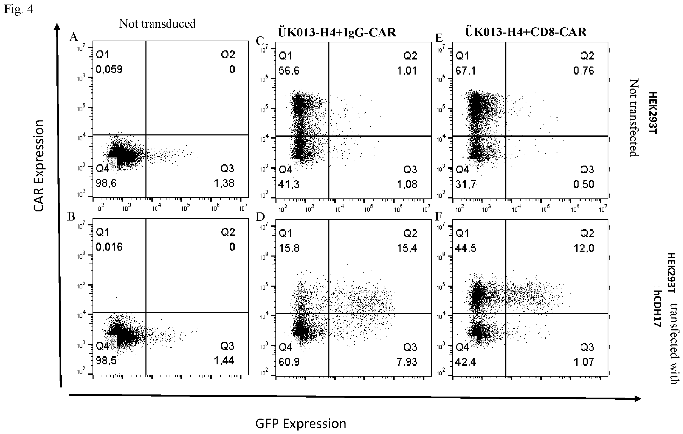

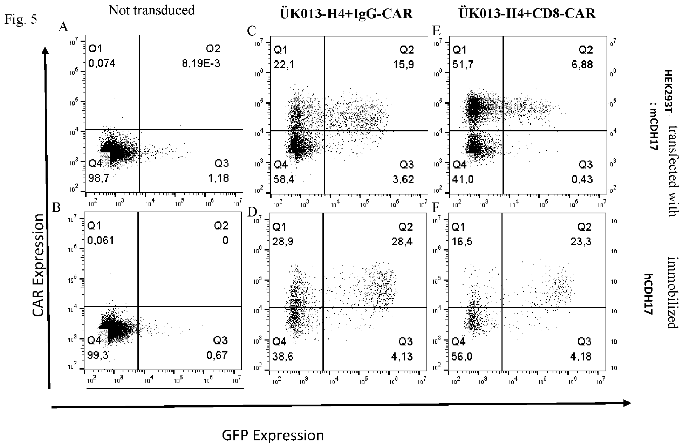

As a representative of a CAR having an scFv specific for human CDH17, a CAR having the scFv ÜK013-H4 as an IgG-CAR and as a CD8-CAR FACS-plots are shown in Fig. 4 and Fig 5. Background detection in reporter cells which were not transduced with a nucleic acid construct encoding a CAR is shown by Figs. 4A and 4B. Fig. 4C and Fig. 4E show that both the IgG-CAR and the CD8-CAR embodiments of the CAR containing the scFv ÜK013-H4 are expressed, and Figs. 4D and 4F show that in presence of HEK293T cells expressing the target antigen hCDH17 the cells expressing the CAR are activated as detected by expression of GFP (GFP Expression) in the reporter cells. The antigen-specific activation is especially shown by Q2, when compared between Fig. 4C (Q2 = 1.01%) without target antigen expressed on cells and Fig. 4D (Q2 = 15.4%) in presence of target antigen expressed on cells, as well as between Fig. 4E (Q2 = 0.76%) without target antigen expressed on cells and Fig. 4F (Q2 = 12.0%) in presence of target antigen expressed on cells.

Fig. 5A and Fig. 5B show background of reporter cells which were not transduced with a nucleic acid construct encoding a CAR. Fig. 5C shows that the IgG-CAR containing the scFv ÜK013-H4 is activated by presence of HEK293T-cells expressing murine CDH17 (mCDH17, Fig. 5C Q2=15.9%), and Fig. 5D shows that this IgG-CAR also in presence of plate-bound human CDH17 (immobilized hCDH17) results in activation of the reporter cells, indicating cross-reactivity for human and murine CDH17. Fig. 5E for the CD8-CAR embodiment containing the scFv ÜK013-H4 shows activation of the reporter cells by murine CDH17 expressed by cells (HEK293T transfected with mCDH17) as the target antigen, although to a lower extent (Fig. 5E, Q2=6.88%) than the embodiment as an IgG-CAR (Fig. 5C, Q2= 15.9%). Fig. 5F shows activation of reporter cells expressing the CD8-CAR embodiment containing the scFv ÜK013-H4 by the antigen immobilized on the culture plate (immobilized hCDH17), also showing cross-reactivity for human and murine CDH17 also for plate-bound antigen.

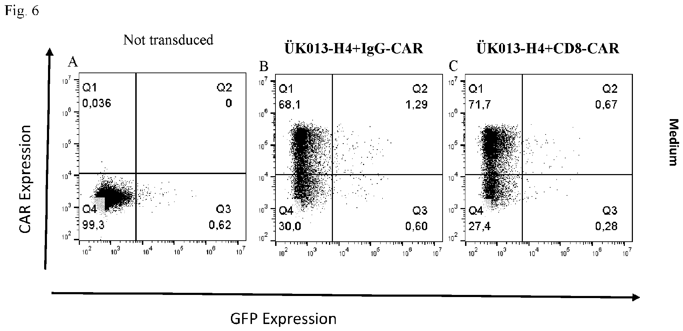

Fig. 6 shows background levels in cell culture medium (Medium) for reporter cells without CAR expression (Fig. 6A, Not transduced), in Fig. 6B for reporter cells transduced for expression of the anti-CDH17-CAR containing the scFv ÜK013-H4 as IgG-CAR, and Fig. 6C for reporter cells transduced for expression of the anti-CDH17-CAR containing the scFv ÜK013-H4 as CD8-CAR, for both these embodiments showing expression of the CAR and very low background activation, indicating the high specificity of the CAR for the antigen.

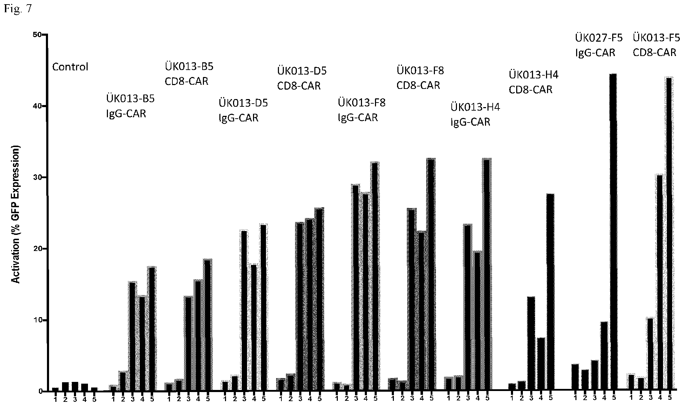

Fig. 7 summarizes background levels and activation levels of reporter cells expressing a CAR as indicated. All CARs of the invention show low background activation in the absence of the target antigen, and all CARs show high activation by presence of the target antigen when expressed on cells or when bound as protein to the culture plate.

## Claims

1. Chimeric antigen receptor (CAR) for use in the treatment of inflammatory bowel disease, the CAR containing from N-terminus to C-terminus an scFv domain, a hinge, a transmembrane domain and at least one intracellular signaling domain (ICD), wherein the scFv domain contains a pair of CDR3 selected from
amino acids No. 121..134 of SEQ ID NO: 2 and amino acids No. 252..260 of SEQ ID NO: 2 (MRU53-F8),
amino acids No. 121..132 of SEQ ID NO: 4 and amino acids No. 250..258 of SEQ ID NO: 4 (MRU53-H1),
amino acids No. 12..132 of SEQ ID NO: 6 and amino acids No. 250..258 of SEQ ID NO: 6 (MRU54-A1),
amino acids No. 119..130 of SEQ ID NO: 8 and amino acids No. 250..259 of SEQ ID NO: 8 (ÜK013-B5),
amino acids No. 119..131 of SEQ ID NO: 10 and amino acids No. 251..260 of SEQ ID NO: 10 (ÜK013-D5),
amino acids No. 118..131 of SEQ ID NO: 12 and amino acids No. 251..260 of SEQ ID NO: 12 (ÜK013-F8),
amino acids No. 119..137 of SEQ ID NO: 14 and amino acids No. 256..266 of SEQ ID NO: 14 (ÜK013-H4),
amino acids No. 119..128 of SEQ ID NO: 16 and amino acids No. 245..253 of SEQ ID NO: 16 (ÜK027-F5).

2. Chimeric antigen receptor for use according to claim 1 or 2, wherein the scFv domain is selected from SEQ ID NO: 1 (MRU53-F8), SEQ ID NO: 3 (MRU53-H1), SEQ ID NO: 5 (MRU54-A1), SEQ ID NO: 7 (ÜK013-B5), SEQ ID NO: 9 (ÜK013-D5), SEQ ID NO: 11 (ÜK013-F8), SEQ ID NO: 13 (ÜK013-H4), SEQ ID NO: 15 (ÜK027-F5).

3. Chimeric antigen receptor for use according to one of the preceding claims, wherein the CAR is encoded by a nucleic acid construct encoding a secretory signal arranged N-terminally to the scFv domain.

4. Chimeric antigen receptor for use according to one of the preceding claims in the treatment of an immune response directed against MEP1A.

5. Chimeric antigen receptor for use according to one of the preceding claims, wherein in a regulatory T-cell the CAR is produced as a fusion protein with FOXP3 with a protease site arranged between the CAR and FOXP3.

6. Chimeric antigen receptor for use according to one of the preceding claims, wherein the hinge is a IgG1 CH1 - IgG1 CH2 - IgG1 CH3 hinge or a CD8 hinge.

7. Chimeric antigen receptor for use according to one of the preceding claims, wherein the CAR between the scFv domain and the hinge contains a linker of 10 to 18 amino acids, in each case ± 2 amino acids.

8. Chimeric antigen receptor for use according to one of the preceding claims, wherein the transmembrane domain is CD4 transmembrane domain or a CD4a transmembrane domain, and the ICD comprises a CD28 signaling domain (CD28 ICD) and a CD3zeta signaling domain (CD3zeta ICD).

9. Regulatory T cell expressing a CAR for use in the treatment of inflammatory bowel disease, wherein the T cell is immunologically compatible with the patient, **characterized in that** the regulatory T cell is genetically manipulated to express a CAR according to one of the preceding claims.

10. Regulatory T cell for use according to claim 9, wherein the T cell is an autologous cell originating from the patient to be treated or the T cell is derived from an autologous cell originating from the patient to be treated.

11. Regulatory T cell for use according to one of claims 9 to 10, **characterized in that** the T cell in the presence of MEP1A or CDH17 has immune suppressive activity.

12. In vitro process for producing a regulatory T cell according to one of claims 9 to 11, **characterized by** introducing into a T cell a nucleic acid construct encoding a CAR according to one of claims 1 to 8.

13. In vitro process according to claim 12, **characterized in that** the nucleic acid construct comprises a portion encoding FOXP3, and the nucleic acid construct is introduced into CD4+ T-cells for converting the CD4+ T-cells into regulatory T-cells expressing the CAR.
